**Europäisches Patentamt**

**European Patent Office**

**Office européen des brevets**

(19)

(11) **EP 1 555 505 A1**

(12) ## DEMANDE DE BREVET EUROPEEN

(43) Date de publication:
**20.07.2005 Bulletin 2005/29**

(51) Int Cl.⁷: **G01B 7/14**, G01D 5/20

(21) Numéro de dépôt: **04075045.7**

(22) Date de dépôt: **16.01.2004**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(71) Demandeurs:
- **Nomics, société anonyme**
  **4000 Liege (BE)**
- **Wallonia Space Logistics Societe Anonyme**
  **4031 Angleur (BE)**

(72) Inventeurs:
- **Ansay, Pierre c/o Nomics, société anonyme**
  **4000 Liège (BE)**
- **Beckers, Bernard c/o Nomics, société anonyme**
  **4000 Liège (BE)**
- **Poirrier, Robert c/o Nomics, société anonyme**
  **4000 Liège (BE)**

(74) Mandataire: **Quintelier, Claude et al**
**Gevers & Vander Haeghen**
**Holidaystraat 5**
**1831 Diegem (BE)**

(54) **Dispositif de mesure de distance**

(57)    Dispositif de mesure de distance comprenant un émetteur et un récepteur, ledit émetteur étant agencé pour produire un champ magnétique à l'aide d'un circuit résonant ayant une fréquence de résonance, ledit récepteur étant agencé pour capter à ladite fréquence de résonance du champ magnétique émis par l'émetteur et convertir l'intensité du champ magnétique capté en un premier signal ayant une valeur d'énergie, ledit émetteur est agencé pour produire ledit champ magnétique par intermittence, chaque émission ayant une énergie prédéterminée, ledit récepteur étant connecté à un détecteur agencé pour déterminer, par corrélation dudit premier signal avec un deuxième signal prédéterminé ayant une forme d'onde représentative d'un signal à capter par le récepteur, un signal de mesure de distance représentant la distance entre l'émetteur et le récepteur. Le dispositif trouve son application dans des détecteurs de troubles de sommeil ou d'autres formes de maladie.

*Fig. 1*

EP 1 555 505 A1

**Description**

L'invention concerne un dispositif de mesure de distance comprenant un émetteur et un récepteur, ledit émetteur étant agencé pour produire un champ magnétique à l'aide d'un circuit résonant ayant une fréquence de résonance, ledit récepteur étant agencé pour capter à ladite fréquence de résonance du champ magnétique émis par l'émetteur et convertir l'intensité du champ magnétique capté en un premier signal ayant une valeur d'énergie.

Un tel dispositif est généralement connu. L'intensité du champ magnétique capté donne une mesure de la distance entre l'émetteur et le récepteur et peut de cette façon être utilisé pour mesurer une distance entre deux points.

L'invention concerne également détecteur de troubles du sommeil comprenant un dispositif de mesure de distance.

Un inconvénient des dispositifs connus est qu'ils ne sont pas adaptés pour mesurer de façon fiable de courtes distances, en particulier inférieures à 10 cm, sans utiliser un champ magnétique d'intensité élevée. Pour cette raison ces dispositifs connus ne sont pas aptes à mesurer des mouvements de la bouche d'un être vivant. En effet des champs magnétiques à puissance élevée ne sont pas appropriés à être utilisés fréquemment sur des êtres vivant sans porter atteinte à la santé de cette être vivant.

L'invention a pour but de réaliser un dispositif de mesure de distance qui est capable de mesurer de façon fiable des courtes distances sans devoir utiliser un champ magnétique puissant.

A cette fin un dispositif suivant l'invention est caractérisé en ce que ledit émetteur est agencé pour produire ledit champ magnétique par intermittence, chaque émission ayant une énergie prédéterminée, ledit récepteur étant connecté à un détecteur agencé pour déterminer, par corrélation dudit premier signal avec un deuxième signal prédéterminé ayant une forme d'onde représentative d'un signal à capter par le récepteur, un signal de mesure de distance représentant la distance entre l'émetteur et le récepteur. L'usage d'une fréquence de résonance et d'une détection à corrélation permet une mesure de distance fiable sans devoir utiliser des champs magnétiques puissant.

L'invention sera maintenant décrite plus en détails à l'aide des dessins qui représentent une forme de réalisation préférentielle d'un dispositif suivant l'invention ainsi que d'un détecteur de troubles du sommeil comprenant un dispositif de mesure de distance suivant l'invention. Dans les dessins :

la figure 1 illustre la structure de base du dispositif de mesure ;
la figure 2 illustre l'excitation de l'émetteur ainsi que le signal émis par l'émetteur et le signal reçu par le récepteur ;
la figure 3 illustre diverses topologies d'un circuit résonant parallèle destiné à exciter l'émetteur ;
la figure 4 illustre deux exemples du circuit du récepteur ;
la figure 5 illustre le principe de la détection par corrélation du signal avec un modèle du signal à détecter ;
les figures 6 et 7 montrent un exemple d'un signal reçu et du signal obtenu après détection par corrélation ;
la figure 8 montre une implémentation numérique de la détection par corrélation ;
la figure 9 montre des modèles utilisables pour la détection par corrélation ;
les figures 10 et 11 montrent les propriétés de rejet de bruit et des parasites de ces trois méthodes de détection par corrélation ;
les figures 12 et 13 montrent le placement du dispositif sur un être vivant ;
les figures 14 a), 14 b) et 15 illustrent à l'aide d'un organigramme une façon de détecter des troubles de sommeil ;
la figure 16 reprend des signaux de mouvement d'une bouche ;
la figure 17 illustre une clef de convolution ;
la figure 18 illustre le résultat de la convolution ;
la figure 19 représente une période de ronflement ;
la figure 20 montre un tableau utiliser pour déterminer les apnées ;et
la figure 21 illustre la position des capteurs pour détecter la maladie de Parkinson.

Dans les dessins une même référence a été attribuée à un même élément ou à un élément analogue.

Le dispositif de mesure de distance utilise un capteur de distance robuste à usage physiologique. Son principe magnétique offre de nombreuses possibilités de mesure, puisqu'il n'est pas influencé par la présence de vêtements ou l'appui contre un oreiller ou un matelas (dans les applications en cours de sommeil), ni par l'état d'agitation ou de transpiration de l'être vivant sur lequel il est appliqué. Le champ magnétique nécessaire à la mesure est extrêmement réduit: l'émission de champ est périodique, à la cadence de mesure, avec un rapport cyclique extrêmement faible, alors que son amplitude maximale atteint des valeurs avec une puissance inférieure à 1mTesla, de préférence inférieure à 1µTesla, voir même de l'ordre de 0,4 µTesla, réputées non nocives pour la santé en cas d'exposition continue.

Le capteur est conçu pour fonctionner en couplage magnétique lâche, ce qui signifie que l'émetteur influence le récepteur, mais que l'atténuation entre l'émetteur et le récepteur ne permet pas au récepteur d'influencer l'émetteur en retour. La méthode de détection est performante et basée sur une détection par corrélation qui permet, malgré le niveau très réduit du champ magnétique émis, d'étendre la gamme de mesure dans un rapport de distance allant jusqu'à 5, voire 6 fois supérieure aux dispositifs connus. Des rapports de distance plus élevés peuvent être atteints en augmentant le champ émis pour limiter l'influence des parasites. Selon les applications, le capteur peut être réalisé avec des composants de très petite taille pour des distances allant de 5 à 30 mm, ou des inductances de taille moyenne (moins de 10mm de diamètre et moins de 15mm de long) pour une gamme de mesure de 4 à 20 cm. Les caractéristiques de ce capteur permettent de réaliser aisément un système ergonomique à placement aisé par le sujet lui-même, de sorte qu'il est utilisable dans les applications ambulatoires.

Plusieurs mesures de distance par rapport à la même référence sont possibles en utilisant un seul circuit d'émission magnétique. Une excitation bilatérale du circuit d'émission magnétique permet en outre d'utiliser ce dispositif pour la transmission d'informations à faible débit et à courte distance. La mesure simultanée de la distance de communication trouve notamment des applications de sécurité. L'obtention de performances plus élevées encore, tant en mesure qu'en fiabilité de transmission, est possible en donnant un caractère pseudo-aléatoire au champ magnétique émis.

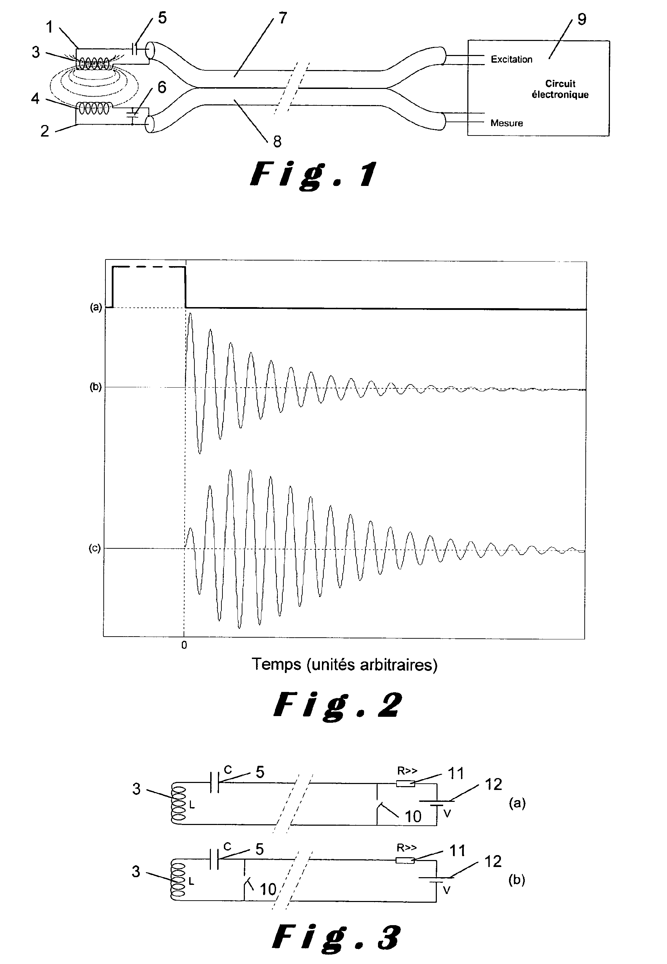

La figure 1 illustre un exemple d'une structure de base d'un dispositif suivant l'invention. Le dispositif comporte un émetteur 1 et un récepteur 2 destinés à être placé à coutre distance l'un de l'autre. L'émetteur comporte une inductance 3 connectée en série avec un condensateur 5, alors que dans le récepteur l'inductance 4 et le condensateur 6 sont connectés en parallèle. L'émetteur 1, respectivement le récepteur 2 sont relié à l'aide d'un câble 7, respectivement 8, à une unité 9 de conditionnement et de mesure. L'unité 9 comprenant un détecteur et un circuit d'excitation comme il sera décrit ci-dessous. Les capteurs utilisés utilisent la propriété qu'a un circuit résonant d'en exciter un autre accordé sur la même fréquence, au travers de leur inductance (3,4) mutuelle. L'utilisation de circuits résonants plutôt que de simples inductances améliore significativement à la fois les performances du circuit d'excitation et la sensibilité du capteur. Pour des questions de confort, les câbles sont généralement rassemblés sur la majeure partie de leur longueur, pour se séparer seulement à l'approche des éléments de mesure.

Afin de limiter le champ magnétique moyen émis, et d'éviter ainsi l'induction à long terme d'effets nocifs ou même simplement gênants pour le patient, et afin de réduire la consommation électrique, le circuit résonant d'émission est excité ponctuellement à chaque prise de mesure, comme illustré à la figure 2. Une impulsion de courant, comme illustrée en 2a, est produite par le circuit d'excitation afin d'exciter l'émetteur à l'aide d'un courant électrique. Le courant circulant dans l'inductance émettrice 3, et qui est proportionnel au champ magnétique émis, prend une forme légèrement différente selon le mode d'excitation adopté, mais d'allure générale telle que représentée à la figure 2b. La tension recueillie au récepteur 2, en cas de compatibilité des circuits résonants, se présente comme à la figure 2c. La résonance se produit suite à la fermeture du circuit résonant au temps 0, après une période d'initialisation préalable montrée en (a), qui a pour but de créer une tension aux bornes de la capacité et/ou un courant dans l'inductance.

L'enveloppe de ce signal dépend du facteur de qualité des circuits résonants utilisés. En cas de mauvaise compatibilité des circuits résonants, cette enveloppe peut se modifier de manière significative, avec l'apparition éventuelle d'un phénomène de battement. La valeur maximale de la tension observée au récepteur varie en fonction de la distance entre inductance d'émission 3 et inductance de réception 4 selon la relation :

$$V = (\alpha/d^3) + \beta \ (1)$$

où à est le gain global de détection, en incluant l'effet des amplificateurs, â un décalage éventuel dû aux circuits de détection et d la distance entre l'émetteur et le récepteur.

Dans la forme préférée de l'invention, les circuits résonants sont accordés sur une fréquence de 5 à 8 kHz, de manière à maximiser le facteur de qualité des circuits résonants tout en restant en-deçà des fréquences radio. L'utilisation d'inductances miniatures peut toutefois nous mener à adopter des fréquences allant jusque 50, voire 100 kHz, en fonction des possibilités du circuit de détection. La valeur et la taille des inductances de résonance sont adaptées de manière à pouvoir baser la détection sur un signal présentant de 10 à 30 périodes de résonance d'amplitude significative. Des durées inférieures dégradent les performances de détection, alors que des valeurs supérieures demandent une précision trop grande, en pratique, dans l'accord des circuits résonants.

Diverses topologies peuvent être adoptées pour le circuit d'excitation. Le choix doit tenir compte du niveau d'excitation désiré (amplitude maximale du champ magnétique), du couplage parasite existant entre les fils 7 et 8 menant de l'unité 9 à l'émetteur et au récepteur (spécialement lors de l'utilisation de câbles très fins pour le confort des personnes), et des aspects de compatibilité électromagnétique. Diverses topologies d'un circuit résonant parallèle sont présentées à la figure 3. Le circuit d'excitation comporte lui une résistance 11, un élément de commutation ou interrupteur 10 et une source de tension 12 branchés en série au travers des conducteurs électriques 7 en série avec l'inductance et le condensateur. L'excitation s'effectue par une fermeture brève de l'interrupteur 10 durant laquelle la capacité 5 est chargée à la tension d'alimentation et un courant est créé dans l'inductance 3. Au moment de l'ouverture de l'interrupteur 10, le circuit formé par l'inductance 3 et la capacité 5 se met donc à osciller sous l'effet de ces deux conditions initiales Le principal attrait de cette méthode est l'obtention aisée de niveaux d'excitation importants ou même variables, en fonction du temps de fermeture de l'interrupteur. Les circuits illustrés à la figure 3 sot des circuits d'excitation série dit « à régénération ». L'excitation est produite par la fermeture de l'interrupteur 10, qui reste fermé tout au long de la mesure. L'ouverture de l'interrupteur permet alors à la capacité 5 de se recharger sans oscillation au travers de la résistance 11.. La forme illustrée à la figure 3a facilite le contrôle de l'interrupteur, alors que la forme 3b permet à la fois d'annuler la tension dans le câble et de rendre constant le courant dans le câble pendant la mesure. Ces formes permettent d'éviter le couplage parasite dans les câbles, puisque l'interrupteur est ouvert durant la mesure. De plus la forme de la figure 3a offre l'avantage que l'interrupteur est plus facile à commander puisqu'il se situe près de la source 12 présente dans l'unité 9.

L'excitation en résonance série peut se réaliser sous deux formes principales, à savoir l'excitation alternée et l'excitation simple à régénération. L'excitation alternée consiste à piloter le circuit résonant par une source de tension d'allure carrée. Une excitation est réalisée à chaque transition de la source de tension, mais le signe du champ électromagnétique émis lors d'une transition descendante est inversé à celui observé lors d'une transition montante. Cet effet doit impérativement être compensé au niveau du circuit de détection pour éviter des imprécisions de mesure. L'excitation simple à régénération consiste à charger lentement la capacité du circuit résonant à travers une résistance de valeur élevée pour éviter une résonance, puis de fermer l'interrupteur pendant toute la mesure pour laisser osciller librement le circuit résonant ainsi formé.

La forme préférée de l'invention utilise donc une excitation à régénération avec une capacité intégrée à l'élément d'émission (figure 3a et b). Ceci permet de maintenir une tension théoriquement nulle tout au long du câble durant la mesure. La topologie à excitation décentralisée (figure 3b) présente en plus l'avantage d'un courant constant durant la mesure.

L'émetteur est agencé pour produire un champ magnétique à l'aide du circuit résonant formé par l'inductance 3 et le condensateur 5. Le circuit résonant possède une fréquence de résonance et le récepteur est agencé pour capter à ladite fréquence de résonance du champ magnétique émis par l'émetteur et convertir l'intensité du champ magnétique capté en un premier signal ayant une valeur d'énergie. Comme illustré à la figure 2 a c'est à partir d'une impulsion fournie au circuit d'excitation que l'émetteur produit le champ magnétique. Ainsi ce champ magnétique est produit par intermittence et chaque émission a une énergie prédéterminée, notamment déterminée par la valeur de l'inductance et du condensateur.

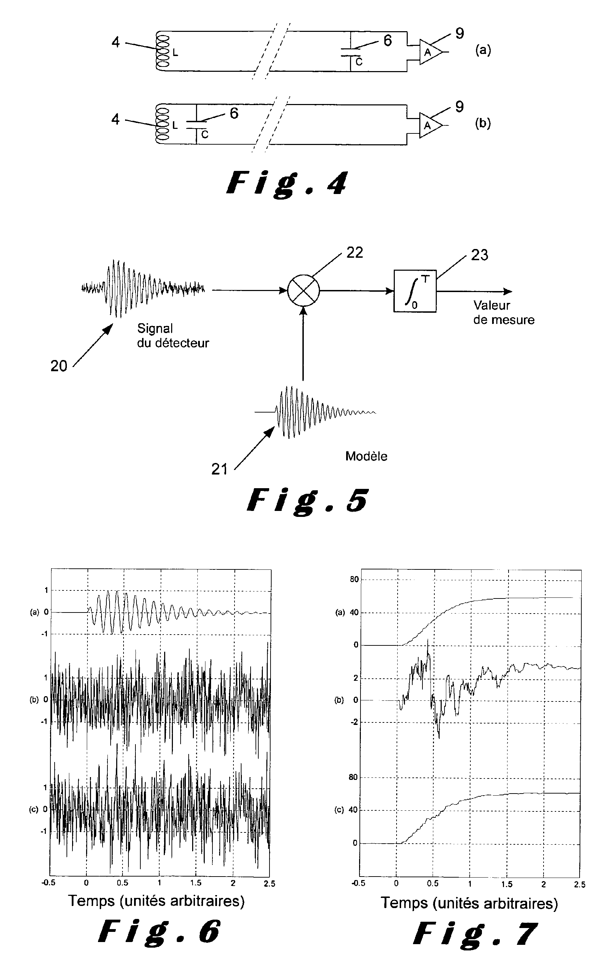

La figure 4 illustre deux exemples du circuit du récepteur 2, notamment (4a) à capacité intégrée et (4b) à capacité éloignée. Le choix de la méthode de détection, c'est à dire d'utilisation de la tension observée pour obtenir une image de la distance, est essentiel pour minimiser les valeurs de champ requises, et ainsi les effets néfastes possibles sur les personnes. La détection est réalisée par corrélation comme il sera décrit ci-dessous.

Si l'on respecte une distance minimale de mesure où le couplage entre les inductances d'émission 3 et de réception 4 est suffisamment lâche, on peut considérer que la forme d'onde du signal reçu n'est modifiée qu'en amplitude en fonction de la distance. On peut donc utiliser l'ensemble de cette forme d'onde pour améliorer la détection. Une méthode procède par l'implémentation d'une détection synchrone. Cette méthode bien connue consiste à multiplier le signal reçu par 1 lorsque la forme d'onde théorique est réputée positive, et par -1 lorsqu'elle est réputée négative, puis d'intégrer le signal ainsi obtenu. Cette détection analogique est représentée sous forme analogique, mais peut tout aussi bien s'effectuer par voie numérique.

Cette détection synchrone est une forme de réalisation d'une détection par corrélation du signal capté par le récepteur avec un deuxième signal prédéterminé ayant une forme d'onde représentative d'un signal à capter par le récepteur. Le résultat issu de cette corrélation constitue alors un signal de mesure de distance représentant la distance entre l'émetteur et le récepteur. La détection par corrélation consiste à multiplier le signal reçu par un modèle, éventuellement modifié, de la forme d'onde théorique, puis d'intégrer le signal ainsi obtenu. La valeur obtenue à la fin du temps d'intégration est la mesure recherchée. Cette méthode générique a l'avantage d'intégrer comme cas particulier les méthodes connues de détection synchrone et de filtrage adapté, selon la forme donnée au modèle de détection. Le principe général est représenté à la figure 5, et son efficacité pour la détection d'un signal bruité est illustrée à la figure 6. Le signal capté 20 est multiplié (22) avec un deuxième signal 21 et le résultat de cette multiplication est intégré (23) pour fournir la valeur de mesure. La figure 6a montre un exemple d'un deuxième signal (21), la figure 6b un exemple du bruit seul, à un niveau relatif, par rapport au signal capté, correspondant à des conditions typiques de détection aux deux tiers de la gamme de mesure de signaux reçus et la figure 6c représente le cas réel où le signal capté est combiné avec le bruit. La figure 7 représente le résultat de l'intégration lors d'une détection par corrélation. Les références a à c dans la figure 7 correspondent à celles de la figure 6. On constate donc dans la figure 7c que le signal de mesure augmente pour atteindre un niveau correspondant à distance entre émetteur et récepteur.

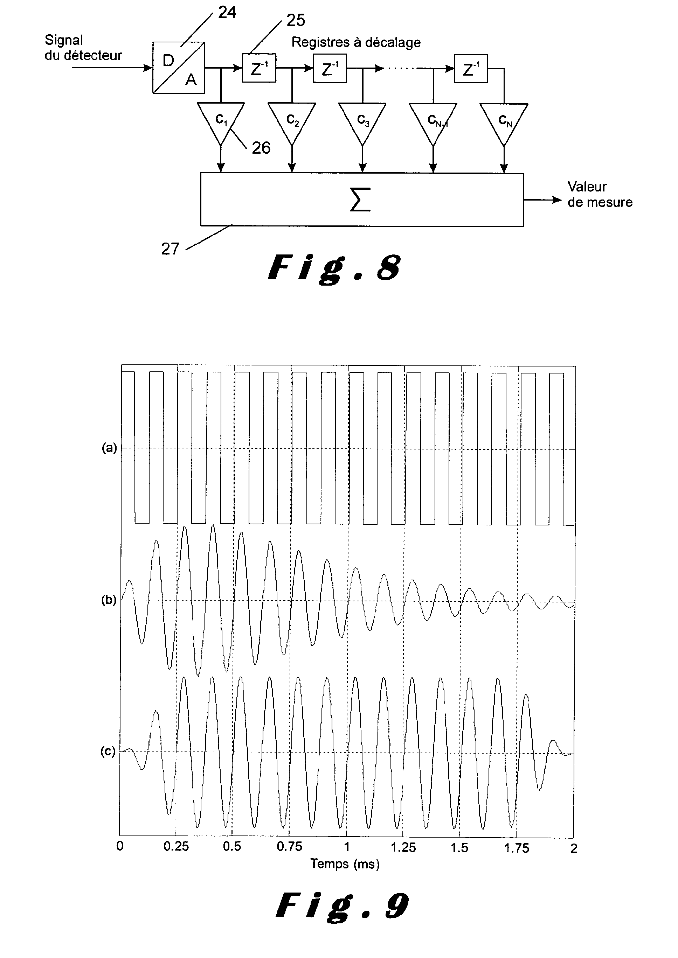

Le filtrage adapté, qui est une forme de réalisation d'un détection par corrélation, est une technique connue en télécommunications est réputée fournir le meilleur rapport signal à bruit sous l'hypothèse d'un bruit blanc gaussien. Des expérimentations ont montré que cette méthode est également excellente dans le cadre de cette invention. Elle est par ailleurs précise si l'on s'interdit l'utilisation de filtres adaptatifs qui posent des problèmes de calibrage des mesures. La technique du filtre adapté consiste à appliquer au signal reçu un filtre dont la réponse pulsionnelle est l'image renversée de la forme d'onde théorique du signal à recevoir, puis d'échantillonner le signal obtenu à un moment précis correspondant à l'alignement parfait du signal utile et du filtre. En d'autres termes, on peut aussi dire que le résultat de la mesure est la valeur de corrélation entre la portion du signal où l'on sait se trouver le signal utile, et la forme d'onde théorique de ce signal utile. Etant donné la complexité de la forme d'onde à réaliser, ce filtrage est réalisé en numérique. L'application numérique de ce principe est représentée à la figure 8. Le signal reçu est converti (24) en numérique après échantillonnage et ses valeurs mémorisées sur N échantillons dans des registres à décalage 25. Ces échantillons sont ensuite multipliés (26) par les coefficients $C_i$ représentant le modèle de détection, et les résultats sont additionnés entre eux pour fournir le signal de mesure. Par intégration dans la présente description on entend donc tant sa signification mathématique que sa réalisation numérique.

La figure 9 montre des modèles utilisables pour la détection par corrélation. Le modèle (fig.9a) du deuxième signal pour une détection synchrone est un signal carré en synchronisation avec le signal capté. Le modèle pour une détection à filtrage adapté (fig.9b) est la forme d'onde théorique elle-même et qui représente le signal à capter tel qu'obtenu en l'absence de perturbations. Le modèle utilisé dans la forme préférée de l'invention est une onde sinusoïdale en synchronisation avec la forme d'onde théorique, multipliée par une fenêtre de Tukey à coefficient d'effilage *(tapper factor)* réduit (typiquement de l'ordre de 0,2 à 0,4) pour adoucir les transitions. Le principe de la fenêtre de Tukey est décrit dans Harris, F.J. « On the use of windows for harmonic analysis with the discrete Fourier transform" et publié dans Proceedings of the IEEE. Vol. 66, January 1978, pages 66 à 67.

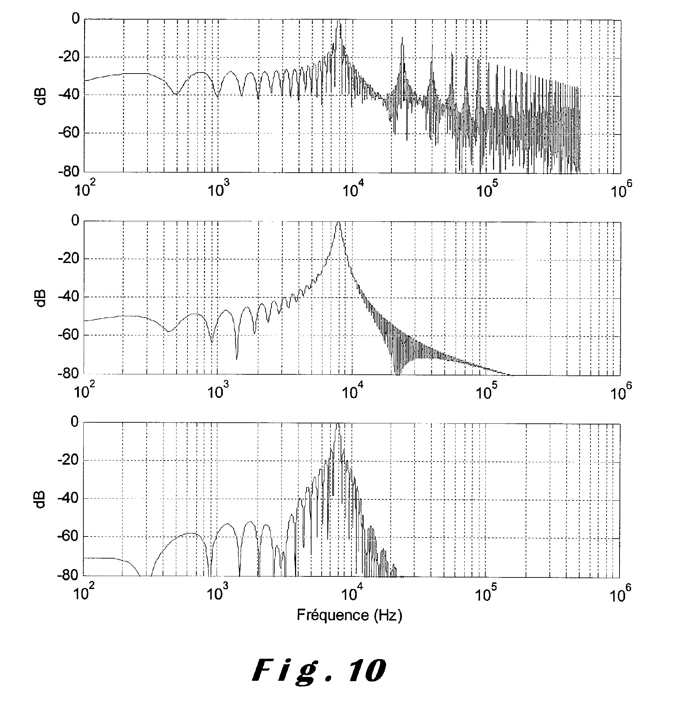

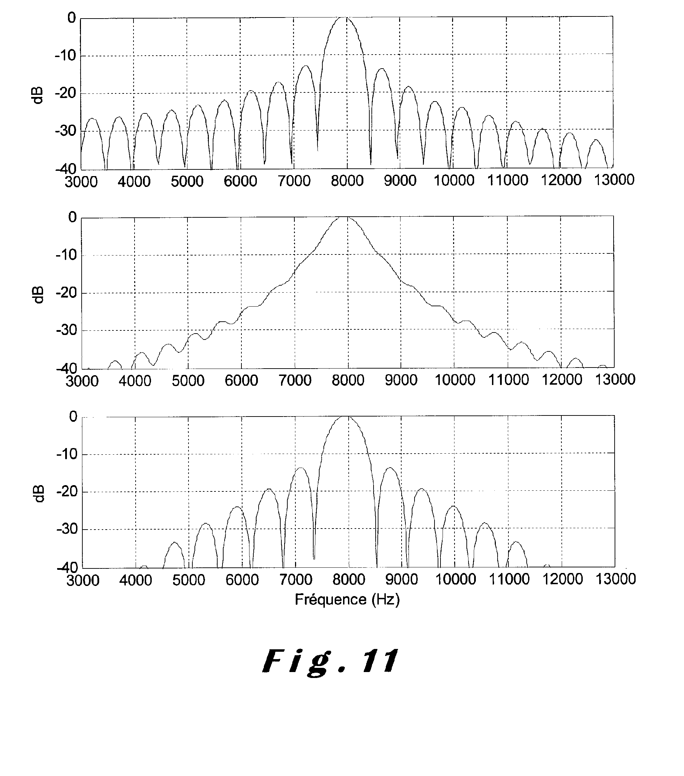

Les propriétés de rejet de bruit et des parasites de ces trois méthodes sont représentées aux figures 10 et 11, respectivement. La figure 10 montre le spectre global de détection des différents modèles de détection : détection synchrone (en haut), filtre adapté (au milieu) et filtre sinusoïdal à fréquence adaptée (en bas).Le filtre adapté se montre plus efficace que la détection synchrone en-dehors de la bande passante, mais moins efficace pour les fréquences proches de celle de résonance. Le modèle sinusoïdal synchrone se montre globalement le plus efficace. La figure 11 montre le spectre de détection aux alentours de la fréquence de résonance des différents modèles de détection : détection synchrone (en haut), filtre adapté (au milieu) et filtre sinusoïdal à fréquence adaptée (en bas).

L'obtention du modèle adéquat pour la détection avec un capteur particulier s'obtient lors d'une procédure de calibrage consistant à placer les deux éléments du capteur à une distance raisonnablement faible, de sorte à obtenir un signal de bonne qualité. La qualité de réception est encore améliorée en prenant la moyenne de ce signal sur plusieurs mesures. La forme d'onde obtenue peut ainsi être utilisée pour dériver, de manière triviale, chacun des modèles de forme d'onde présentés.

Performances obtenues avec un capteur optimisé pour un fonctionnement entre 2,5 et 10 cm :

| Distance de référence | 30 mm | 50 mm | 80 mm | 100 mm |
|---|---|---|---|---|
| Précision | 0,3 % | 0,5 % | 0,2 % | 0,1 % |
| Ecart-type dû au bruit | 0,003 mm | 0,05 mm | 0,30 mm | 0,73 mm |

Le stockage des formes d'onde du filtre adapté dans une mémoire placée dans le connecteur du capteur est utilisé pour rendre indépendantes le calibrage du capteur et du circuit de mesure, et faire ainsi en sorte que n'importe quel capteur (lors d'un remplacement, par exemple) puisse être branché sur le circuit en obtenant les mêmes résultats. Une autre méthode de stockage est dans la mémoire du microcontrôleur placé sur le circuit de mesure. Mais dans ce cas, il faut refaire un calibrage à chaque fois que l'on change de capteur.

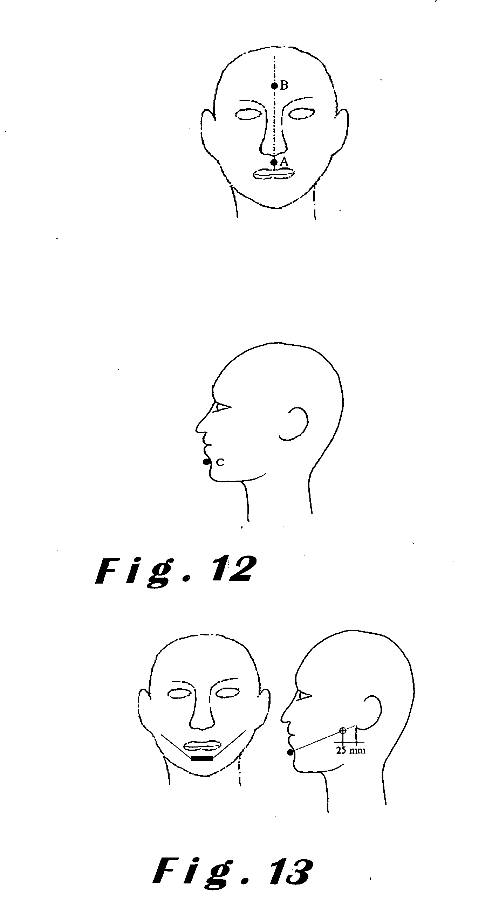

Une application du dispositif de mesure suivant l'invention est celle à un détecteur de troubles de sommeil. Le dispositif est alors monté sur un support agencé pour être appliqué sur la tête d'un être vivant de telle façon à mesurer des mouvements de la bouche ; Les paramètres de fonctionnement du capteur sont ajustés pour produire un bruit de mesure crête-à-crête inférieur à 1 mm pour des ouvertures de bouche jusqu'à 5cm par rapport à la position bouche fermée. La référence de la mesure peut être placée n'importe où le long de la ligne médiane du visage, au-dessus de la lèvre supérieure, comme indiqué à la figure 12. Une réalisation courante de l'invention place cette référence sous le nez (A).

Une autre réalisation courante place cette référence sur le front (B). Alternativement, le point de référence peut être placé à l'intérieur de la bouche, sur les dents, le palais ou les gencives. Le point de mesure du capteur doit être placé de manière à suivre au mieux les mouvements de la mandibule. Dans la réalisation préférée de l'invention, il est placé dans le creux sous la lèvre inférieure (figure13), là où les mouvements relatifs entre la structure osseuse et la peau sont les plus faibles. Alternativement, le point de mesure peut être situé à la pointe du menton, sous le menton, ou à l'intérieur de la bouche, sur les dents ou les gencives. Dans la forme préférée de l'invention, les deux éléments du capteur sont maintenus en place au moyen d'un harnais confortable, peu encombrant et de placement aisé. Le maintien du point de référence peut être effectué par n'importe quel moyen connu. Le maintien du point de mesure est plus délicat. Pour minimiser la gêne pour le patient, les structures situées près de la bouche et sur les joues doivent être très légères. D'autre part, il convient d'assurer un maintien efficace sur toute la dynamique d'ouverture sans imposer d'effort sur la mandibule qui risquerait d'influencer les mesures. Enfin, une indépendance vis-à-vis des mouvements de rotation et d'inclinaison de la tête vers l'avant ou vers l'arrière est indispensable. La forme préférée de l'invention présente une structure de capteur de forme allongée munie d'un lien imposant une faible traction depuis le creux situé sous la lèvre inférieure le long d'une ligne passant sous le lobe des oreilles, avec un point de fixation situé 25mm au moins en avant du lobe. Alternativement, un maintien complémentaire du capteur peut être assuré par une mentonnière.

Une autre possibilité de placement consiste à placer un capteur sur les paupières et un autre à proximité de l'oeil. Le but étant de mesurer les mouvements des paupières (technique utilisée également pour la détection de la somnolence). Pour étudier le Periodic Limb Movement (PLM) le capteur est placé de part et d'autre d'une articulation. Le but étant de mesurer les variations de distance de cette articulation. Une autre application serait de dire que le capteur peut être utilisé pour la transmission de données à courte distance.

La détection des troubles respiratoires du sommeil est une des applications du dispositif suivant l'invention. La surveillance des troubles respiratoires en sommeil s'applique également au cas de patients sous traitement par pression d'air positive. Ici, l'analyse réalisée du signal d'ouverture de la bouche est utilisé pour ajuster en temps réel la valeur de la pression fournie par l'appareil de PAP.

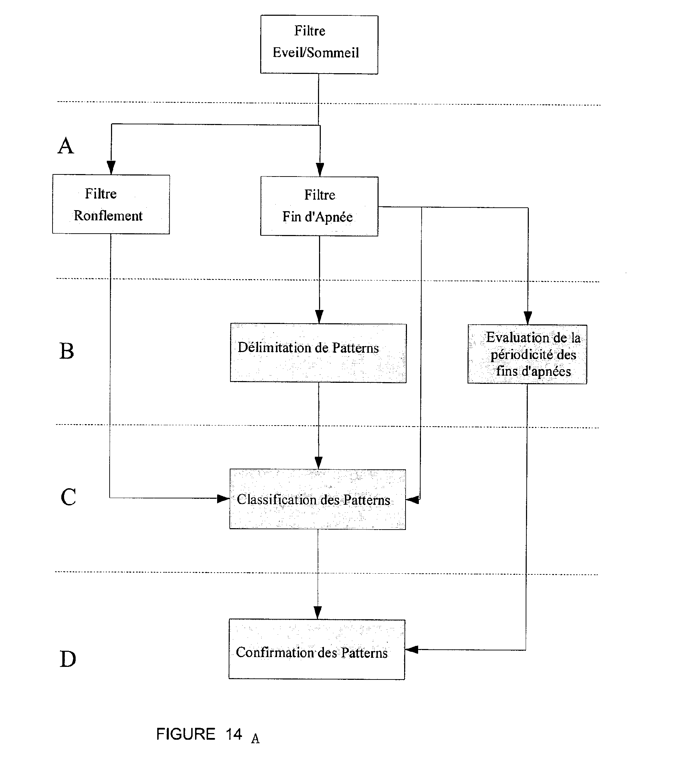

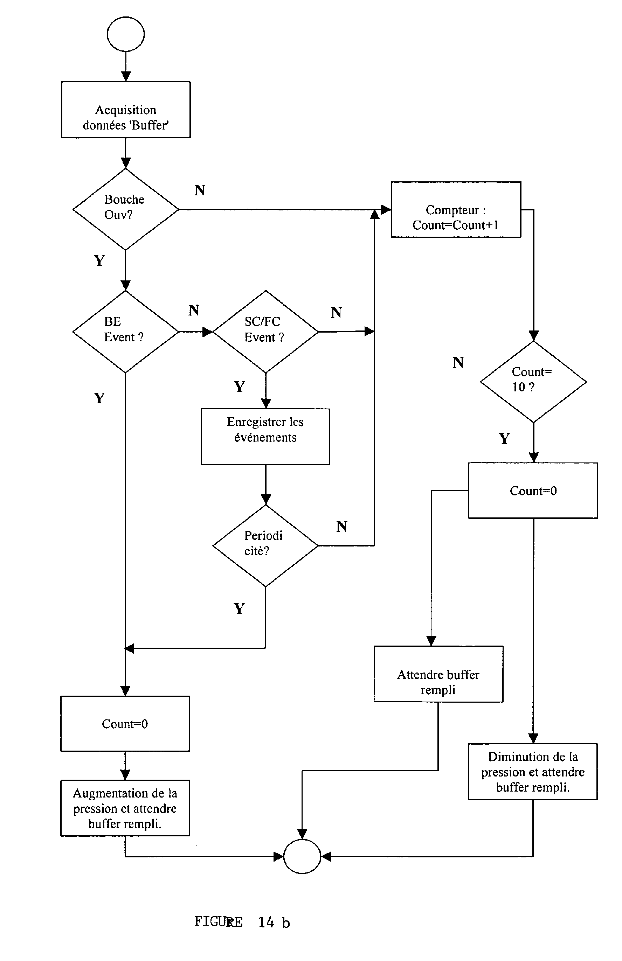

Dans cette application, des techniques de traitement de signal sont utilisées de manière à détecter tous les troubles respiratoires du sommeil. On démarre avec les données des capteurs et dérivons, par filtrage, des signaux supplémentaires. C'est la combinaison de ces signaux qui révèle les différents troubles respiratoires du sommeil: apnée centrale (ou cheynes-stokes), apnée mixte, apnée obstructive, hypopnée et résistance accrue de la voie aérienne supérieure (RAVAS ou en anglais UARS - upper airways resistance syndrome). Le diagramme schématique est décrit de ces troubles respiratoires du sommeil sont illustrés aux figures 14 a), 14 b) et 15. Le but de l'analyse est donc de déterminer si la pression appliquée est suffisante, correcte ou exagérée. Cette information est utilisée pour déterminer les ajustements de pression nécessaire et les envoyer à la CPAP.

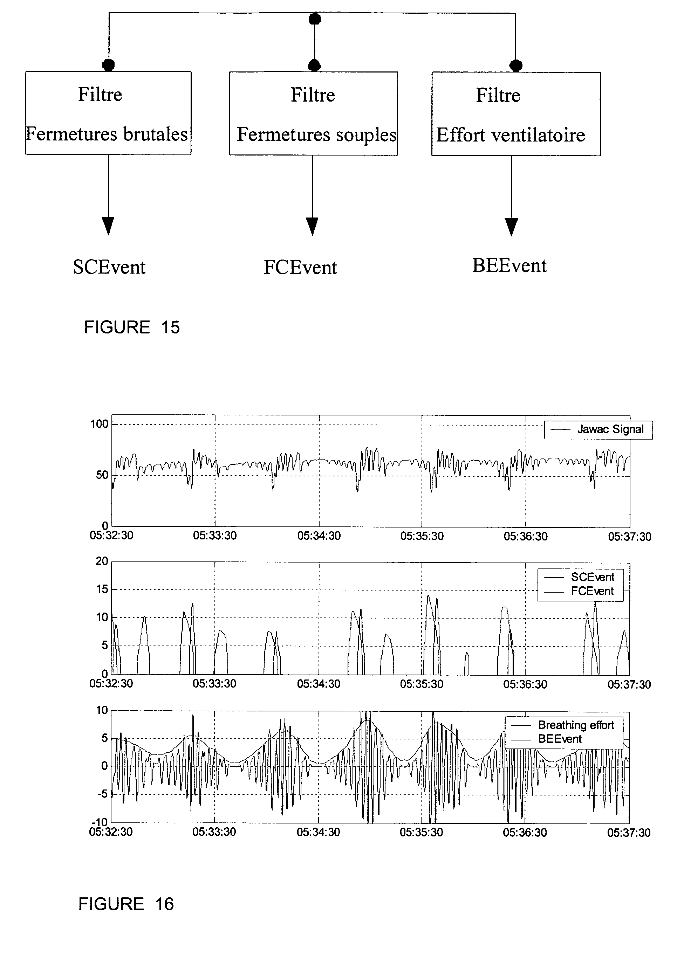

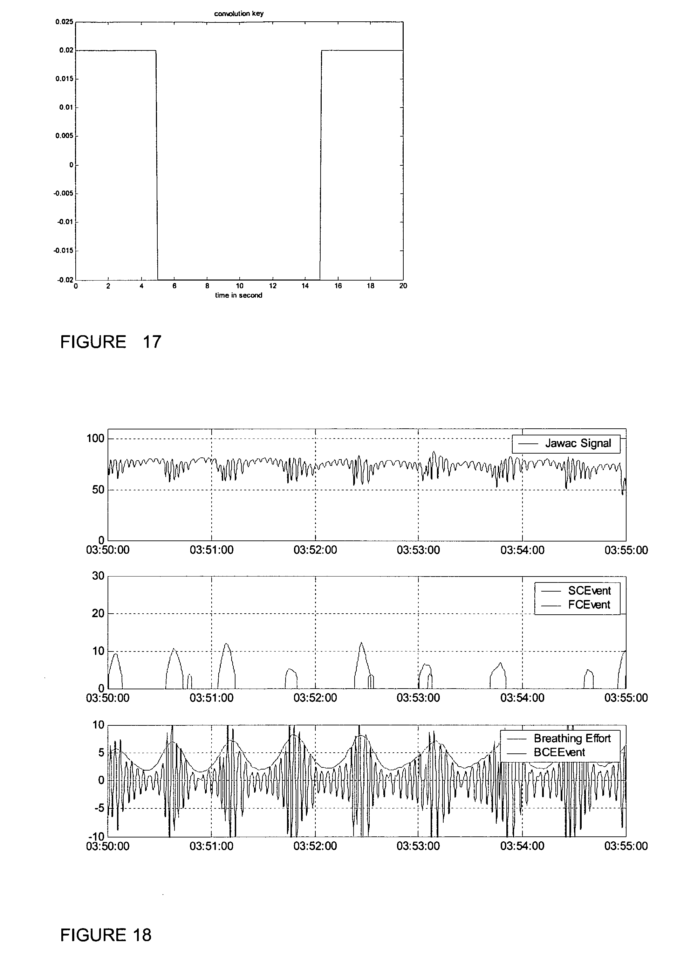

L'objectif de cette première étape (A) est d'utiliser des techniques de filtrage qui permettent de révéler les caractéristiques essentielles des apnées. Ces étapes sont précisées à la figure 15. Ces caractéristiques sont connues et décrite dans la thèse du Prof. R. Poirrier. La sortie de chaque filtre est également un signal contenant des événements. Un événement est marqueur d'une caractéristique potentielle d'une apnée et c'est la combinaison des ces événements qui sera utilisée pour la classification des différents types d'apnée. La figure 16 illustre cette méthode. Le signal de mesure de distance obtenu à partir des capteurs est représenté dans la première partie de la figure dans une fenêtre temporelle de cinq minutes. Ici, la valeur cent signifie que la mandibule est complètement fermée et le zéro signifie quant à lui que la mandibule est grande ouverte.

Le signal des événements de fermetures brutales (SCEvent - Sudden Closing Event) est un signal obtenu en calculant la différence de moyenne du signal de mesure. Le signal SCEvent n'est que la partie positive des résultats de la différence de moyenne mettant en évidence les endroits où la mandibule est fermée brutalement. L'amplitude de se signal dérivé révèle la grandeur de cette fermeture. Ce signal est, par conséquent, un marqueur d'événement de fermetures brutales.

Le signal des événements de fermetures souples (FCEvent - Fluid Closing Event) est un signal obtenu par convolution du signal de mesure et d'une clé de convolution. Cette clé de convolution est reprise à la figure 17. L'objectif de cette convolution est de mettre en évidence les variations souples du signal de mesure qui représentent une ouverture de la mandibule suivie par une fermeture opposée et similaire. Le résultat de la convolution est repris à la figure 18 (FCEvent).

Le dernier signal obtenu par filtrage est le signal BCEvent. Nous filtrons tout d'abord le signal de mesure avec un filtre passe bande dans la bande de fréquence de la respiration (typiquement 0.2 à 0.4 Hz). Le résultat du filtrage est ce qui est appelé le signal d'effort respiratoire (Breathing Effort). Dès lors, le signal SCEvent est simplement l'enveloppe supérieure du signal d'effort respiratoire.La totalité de ces trois signaux dérivés sont les marqueurs d'une fin possible d'un événement apnéique.

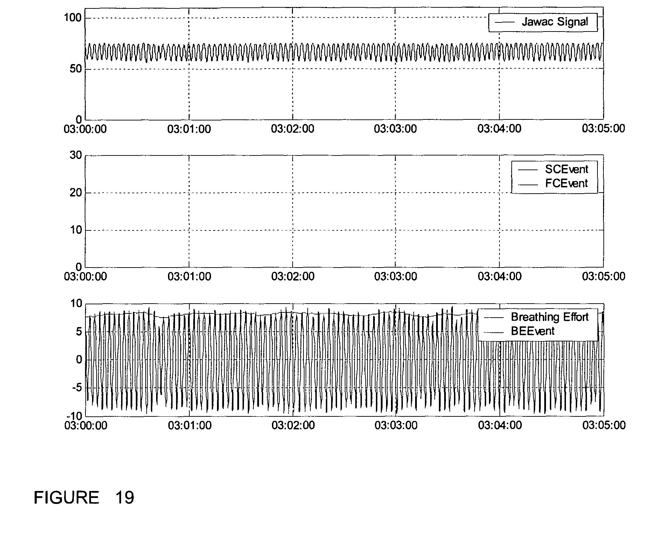

Le second objectif de cet étage A (figure14) est de détecter les périodes de ronflement. Le ronflement est défini comme un signal sinusoïdale oscillant à la fréquence de la respiration (typiquement 0.2 ... 0.4 Hz) et d'amplitude constante. La figure 19 représente une période de ronflement.

L'étape suivante de l'algorithme est utilisée pour délimiter tous les événements apnées candidats. La durée d'une apnée est généralement définie dans une fenêtre temporelle de 10 à 90 secondes. Les trois signaux dérivés (SCEvent, FCEvent, BEEvent) sont tout d'abord utilisés pour rejeter les événements qui ne représentent pas des apnées. Un événement est accepté si une des deux conditions suivantes est remplie:

- La présence de SCEvent et BEEvent dans la fenêtre temporelle considérée.
- La présence de FCEvent et BEEvent dans la fenêtre temporelle considérée.

Ensuite, pour tous les événements acceptés, il est regardé en arrière dans la fenêtre temporelle considérée pour chercher où l'apnée est supposée avoir débuté. Typiquement, l'apnée commence quand l'événement BBEvent commence à augmenter dans la période de temps considérée. C'est le cas pour les hyponées et les apnées obstructives. Dans le cas des apnées centrales ou mixtes, l'apnée commence avec aucun effort respiratoire et l'événement BBEvent est, dans ce cas, la signature de la fin de l'apnée. Il faut, par conséquent, dériver un autre signal de manière à délimiter les apnées centrales et mixtes. Dans la période temporelle considérée, on marque les endroits où l'effort respiratoire est nul. Le signal des événements NEBEvent pointe l'absence d'effort respiratoire en début d'apnée.

Le dernier signal dérivé est utilisé pour la détection des syndromes de résistance accrue de la voie aérienne supérieure (SRAVAS ou en anglais UARS - upper airway resistance syndrome): Dans la fenêtre temporelle considérée d'une apnée, nous marquons si l'ouverture moyenne de la mandibule est linéaire durant la totalité de l'apnée.

Le second aspect important de cette étape est de trouver les portions de signal où un des trois événements ci-dessus se répète avec une période temporelle stricte. La périodicité d'un événement sera utilisée pour confirmer la présence d'une salve d'apnée.

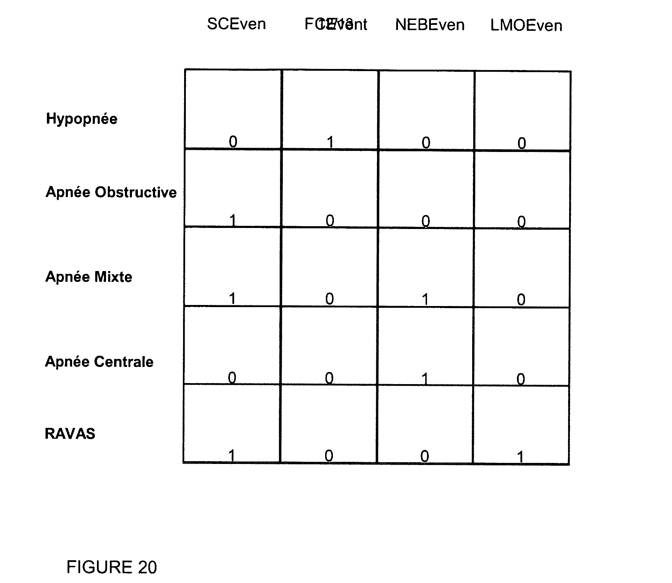

Le but de cette étape est de classifier toutes les apnées délimitées. Nous utilisons la table binaire suivante. Pour chaque apnée délimitée, le type d'apnée est caractérisé par la présence ou l'absence de tous les événements dans la région délimitée. Le tableau repris à la figure 20 illustre ceci.

Deux règles sont utilisées pour confirmer la classification ci-dessus. La classification ci-dessus peut contenir des grapho-éléments qui ne sont pas à proprement parlé des apnées. De manière à obtenir une spécificité et une sensibilité suffisante de l'algorithme, on accepte uniquement les grapho-éléments qui satisfont au moins une des deux règles suivantes :

Règle 1: Toutes les apnées détectées dans une région 'périodique' seront acceptées.
Règle 2: Chaque grapho-éléments classé qui contient un événement BEEvent supérieur à un seuil spécifié sera accepté. Ici, les apnées isolées qui sont détectées mais qui n'ont pas un effort respiratoire suffisant seront rejetées.

De façon similaire à la détection des troubles respiratoires du sommeil,on utilise les capteurs pour réguler la pression d'un appareil à pression d'air positif et continu (continuous positive air pressure apparatus).

Le principe de la méthode proposée est le suivant :

1) On collecte les données dans un tampon. La longueur de celui-ci est définie de manière à contenir au moins une apnée. (typiquement 10 à 90 s).
2) On vérifie tout d'abord que la bouche est plus ou moins fermée (typiquement 80%).
3) Si la bouche est ouverte, on vérifie la présence des événements suivants : BEEvent, SCEvent et FCEvent. La périodicité est également vérifiée pour confirmation. Si un tel événement est observé, on augmente la pression et attend l'ensemble de données suivantes.
4) Si la bouche est plus ou moins fermée (habituellement 85 % de fermeture), On donne la possibilité d'utiliser une échelle temporelle différente pour diminuer la pression. La diminution de pression est plus lente que l'augmentation. Ici, on considère que la diminution de pression est dix fois plus lente que l'augmentation. Le rapport entre l'augmentation et la diminution peut être adapté pour chaque personne.

Le même détecteur de distance peut être utilisé dans tout autre application nécessitant la surveillance ou l'analyse du mouvement d'une articulation, en plaçant les deux éléments du capteur de part et d'autre de celle-ci.

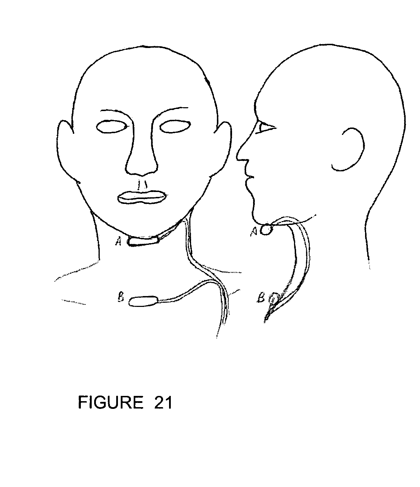

Le suivi de l'évolution de la maladie de Parkinson chez un patient en est un exemple. Dans ce cadre, il est utile d'enregistrer durant un ou plusieurs jours, l'état du patient pour détecter des situations de tremblement, de figement et de repliement. A cet effet, le capteur de distance peut être placé au niveau du menton et du thorax, comme indiqué à la figure 21, afin de mesurer les caractéristiques des mouvements de la tête. Des statistiques peuvent alors être établies pour juger de l'efficacité d'un traitement, par exemple. Alternativement, les capteurs peuvent être placés au coude, à la hanche, ou au genou avec des effets similaires.

- L'état de figement est détecté par une analyse de variabilité du signal sur des périodes données. On peut par exemple procéder par calcul de la variance du signal. Une variance de faible valeur est représentative d'un état de figement.
- L'état de repliement est un état de figement où la valeur moyenne de distance mesurée est de plus, très faible.
- Un état de tremblement peut être détecté par une analyse spectrale du signal. L'apparition d'une composante spectrale prédominante dans le signal de distance sur une période donnée est représentative d'un état de tremblement.

Le mouvement périodique des membres est une autre maladie du sommeil. L'utilisation du même détecteur de distance pour la détection de ces mouvements est possible. Le principe est tout à fait semblable au cas du suivi de la maladie de Parkinson. Toutefois, l'analyse recherche plutôt des mouvements isolés et leur périodicité plutôt que des états généraux.

La littérature abonde sur les moyens de détecter la perte de vigilance d'une personne, notamment durant la conduite d'un véhicule. Le capteur de distance présenté ici est utilisé pour de telles mesures selon deux modalités:

a. Mesure du dodelinement de la tête

L'apparition d'un état de somnolence s'accompagne d'un relâchement des muscles du cou qui se traduit, en position verticale, par un abaissement progressif de la tête. Lorsque la personne en prend conscience, elle réagit en redressant brusquement la tête. Le placement du capteur de distance sur le menton et sur le thorax, conformément à la figure 21, permet de détecter ces événements. L'analyseur procède donc par repérage des mouvements brusques vers le haut, ce qui constitue un cas particulier de l'analyse.

b. Mesure de l'ouverture de la paupière

La miniaturisation des composants permet de placer un des éléments du capteur de distance sur la paupière, l'autre étant placé sous ou au-dessus de l'oeil. Lors d'une perte progressive de vigilance, de battements de paupières plus fréquents sont observés, de même qu'une diminution progressive de l'ouverture moyenne de la paupière.

La détection de ces événements constitue également un cas particulier de l'analyse.

Des applications de sécurité nécessitent le contrôle du déclenchement d'une machine ou d'un dispositif en fonction de la distance, de telle manière à éviter une fausse manoeuvre ou éviter un usage intempestif. L'utilisation de ce capteur de distance à excitation ponctuelle permet cette utilisation. Dans ce cas, l'interrupteur du circuit d'excitation peut servir à la demande de déclenchement de l'utilisateur, mais le déclenchement effectif ne sera réalisé que par un élément matériel ou logiciel de décision après vérification de la distance à laquelle se trouve le dispositif de déclenchement. Un déclenchement de différents dispositifs en fonction de la distance est également possible, par exemple, mais non seulement, pour afficher des messages d'avertissement différents.

## Revendications

1. Dispositif de mesure de distance comprenant un émetteur et un récepteur, ledit émetteur étant agencé pour produire un champ magnétique à l'aide d'un circuit résonant ayant une fréquence de résonance, ledit récepteur étant agencé pour capter à ladite fréquence de résonance du champ magnétique émis par l'émetteur et convertir l'intensité du champ magnétique capté en un premier signal ayant une valeur d'énergie, **caractérisé en ce que** ledit émetteur est agencé pour produire ledit champ magnétique par intermittence, chaque émission ayant une énergie prédéterminée, ledit récepteur étant connecté à un détecteur agencé pour déterminer, par corrélation dudit premier signal avec un deuxième signal prédéterminé ayant une forme d'onde représentative d'un signal à capter par le récepteur, un signal de mesure de distance représentant la distance entre l'émetteur et le récepteur.

2. Dispositif de mesure de distance suivant la revendication 1, **caractérisé en ce que** ledit détecteur est agencé pour réaliser ladite corrélation par multiplication et intégration avec ledit deuxième signal, lequel deuxième signal est formé par ladite forme d'onde représentant le signal à capter tel qu'obtenu en l'absence de perturbation.

3. Dispositif de mesure de distance suivant la revendication 1, **caractérisé en ce que** ledit détecteur est agencé pour réaliser ladite corrélation par multiplication et intégration avec ledit deuxième signal, lequel deuxième signal est formé par ladite forme d'onde représentant une forme d'onde sinusoïdale en synchronisation avec le premier signal elle-même multipliée par une fenêtre de Tukey à coefficient d'effilage réduit.

4. Dispositif de mesure de distance suivant la revendication 1, **caractérisé en ce que** ledit détecteur est agencé pour réaliser ladite corrélation par multiplication et intégration avec ledit deuxième signal, lequel deuxième signal est formé par ladite forme d'onde représentant une forme d'onde carrée en synchronisation avec le premier signal.

5. Dispositif de mesure de distance suivant l'une des revendications 1 à 4, **caractérisé en ce que** ledit émetteur est logé dans un boîtier et agencé pour produire ledit champ magnétique à l'extérieur dudit boîtier avec une puissance inférieure à 1mTesla, de préférence inférieure à 1μTesla.

6. Dispositif de mesure de distance suivant l'une des revendications 1 à 5, **caractérisé en ce que** ledit émetteur comporte une inductance et un condensateur connectés en série entre eux et raccordés à l'aide de conducteurs électriques à un circuit d'excitation, ledit circuit d'excitation comprenant une source de tension et une résistance connectées, au travers desdits conducteurs électriques, en série avec l'inductance et le condensateur, ledit circuit d'excitation comprenant également un élément de commutation permettant de connecter entre eux lesdits conducteurs électriques.

**7.** Dispositif de mesure de distance suivant la revendication 6, **caractérisé en ce que** ledit circuit d'excitation est placé à proximité de l'inductance et du condensateur et forme une unité autonome par rapport au récepteur.

**8.** Détecteur de troubles du sommeil comprenant un dispositif de mesure de distance suivant l'une des revendications 1 à 7, **caractérisé en ce que** ledit dispositif est monté sur un support agencé pour être appliqué sur la tête d'un être vivant de telle façon à mesurer des mouvements de la bouche.

**9.** Détecteur de troubles du sommeil suivant la revendication 8, **caractérisé en ce que** ledit détecteur comporte un analyseur ayant une entrée reliée au dispositif et agencée pour recevoir ladite mesure de distance, ledit analyseur étant agencé pour fractionner ledit signal de mesure de distance et appliquer des fenêtres temporelles sur chaque fraction du signal de mesure de distance ainsi obtenu, ledit détecteur comprenant également une mémoire pour stocker une série de formes de signaux caractérisant dans une fenêtre temporelle des mouvements de la bouche d'un être vivant, ledit analyseur étant agencé pour comparer lesdites fractions du signal de mesure de distance à chacune desdites formes de la série et pour produire un signal de détection en cas de correspondance entre ladite fraction et ladite forme, le signal de détection comprenant également un indicateur indiquant la forme ayant menée à ladite correspondance.

**10.** Détecteur de troubles du sommeil suivant la revendication 9, **caractérisé en ce que** ladite série comporte une première forme indiquant une fermeture soudaine de la bouche, une deuxième forme indiquant une lente ouverture de la bouche suivie d'une lente fermeture de la bouche ainsi qu'une troisième forme indiquant un accroissement de l'amplitude du signal à la fréquence de respiration suivie d'une décroissance du signal à la fréquence de respiration.

**11.** Détecteur de troubles du sommeil suivant la revendication 9, **caractérisé en ce que** ledit analyseur est agencé pour produire un signal d'apnée lorsque pour une même fenêtre le signal de détection indique à la fois une première et une troisième forme ou indique à la fois une deuxième et une troisième forme.

**12.** Détecteur de troubles du sommeil suivant l'une des revendications 9 à 11, **caractérisé en ce que** ladite série comporte une quatrième forme indiquant un ronflement.

**13.** Détecteur de troubles du sommeil suivant l'une des revendications 9 à 12, **caractérisé en ce que** ledit détecteur comporte un analyseur ayant une entrée reliée au dispositif et agencée pour recevoir ledit signal de mesure de distance, ledit analyseur étant agencé pour repérer dans ledit signal de mesure de distance des formes de signaux représentant des événements brefs et récurrents et pour produire un signal de détection à chaque occurrence de tels signaux, le signal de détection comprenant également un indicateur indiquant la forme ayant menée audit signal de détection.

**14.** Détecteur de troubles du sommeil suivant la revendication 13, **caractérisé en ce qu'**il comporte un élément de décision utilisant ledit signal de détection pour fournir une indication de traitement insuffisant, correct ou exagéré des troubles du sommeil visés.

**15.** Analyseur de mouvement comprenant un dispositif de mesure de distance suivant l'une des revendications 1 à 8, **caractérisé en ce que** ledit dispositif est monté sur un support agencé pour être appliqué autour d'une articulation d'un être vivant de telle façon à mesurer les caractéristiques et/ou les statistiques des mouvements de cette articulation.

**16.** Détecteur de mouvements périodiques des membres en cours de sommeil comprenant un analyseur de mouvement suivant la revendication 15, **caractérisé en ce que** ledit analyseur est agencé pour repérer dans ledit signal de mesure de distance des formes de signaux représentant des événements brefs et récurrents et pour produire un signal de détection à chaque occurrence de tels signaux, le signal de détection comprenant également un indicateur indiquant la forme ayant menée audit signal de détection.

17. Appareillage de suivi de l'évolution de la maladie de parkinson comprenant un analyseur de mouvement suivant la revendication 15, **caractérisé en ce que** ledit analyseur est agencé pour repérer dans ledit signal de mesure de distance des formes de signaux représentant des états de tremblement, de figement et de repliement pour produire un signal de détection, le signal de détection comprenant également un indicateur indiquant la forme ayant menée audit signal de détection.

18. Appareillage de détection d'une perte de vigilance comprenant un analyseur de mouvement suivant la revendication 15, **caractérisé en ce que** ledit dispositif de mesure est placé de telle manière à mesurer l'inclinaison de la tête et ledit analyseur est agencé pour repérer dans ledit signal de mesure de distance des événements d'inclinaison lente et relève brutale de la tête, pour produire un signal de détection.
Appareillage de détection d'une perte de vigilance comprenant un analyseur de mouvement suivant la revendication 15,**caractérisé en ce que** ledit dispositif de mesure est agencé de telle manière à mesurer l'amplitude d'ouverture des paupières et ledit analyseur est agencé pour repérer dans ledit signal de mesure de distance des événements de clignement récurrent des paupières et un état de diminution progressive de l'amplitude moyenne d'ouverture des paupières, pour produire un signal de détection.

EP 1 555 505 A1

**Fig. 1**

Temps (unités arbitraires)

**Fig. 2**

**Fig. 3**

11

**Fig. 4**

Signal
du détecteur

Modèle

Valeur
de mesure

**Fig. 5**

Temps (unités arbitraires)

**Fig. 6**

Temps (unités arbitraires)

**Fig. 7**

**_Fig. 8_**

**_Fig. 9_**

**Fig. 10**

*Fig. 11*

**Fig. 12**

**Fig. 13**

A

**Filtre Eveil/Sommeil**

**Filtre Ronflement**

**Filtre Fin d'Apnée**

B

**Délimitation de Patterns**

**Evaluation de la périodicité des fins d'apnées**

C

**Classification des Patterns**

D

**Confirmation des Patterns**

FIGURE 14 A

FIGURE 14 b

FIGURE 15

FIGURE 16

FIGURE 17

FIGURE 18

FIGURE   19

|  | SCEven | FCEvent | NEBEven | LMOEven |
|---|---|---|---|---|
| **Hypopnée** | 0 | 1 | 0 | 0 |
| **Apnée Obstructive** | 1 | 0 | 0 | 0 |
| **Apnée Mixte** | 1 | 0 | 1 | 0 |
| **Apnée Centrale** | 0 | 0 | 1 | 0 |
| **RAVAS** | 1 | 0 | 0 | 1 |

FIGURE 20

FIGURE 21

**Office européen**
**des brevets**

**RAPPORT DE RECHERCHE EUROPEENNE**

Numéro de la demande

EP 04 07 5045

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | DE 41 14 398 A (LETRON GMBH ELECTRONIC LEHESTE) 29 octobre 1992 (1992-10-29) | 1,4,5 | G01B7/14 G01D5/20 |
| A | * colonne 1, ligne 30 - colonne 2, ligne 49; figures 1,2 *<br>--- | 2,3,6-19 | |
| A | US 4 665 361 A (DORSCH MANFRED ET AL) 12 mai 1987 (1987-05-12) * colonne 2, ligne 46 - colonne 4, ligne 39; figures 1-4 *<br>--- | 1-19 | |
| A | US 4 843 259 A (WEISSHAUPT BRUNO) 27 juin 1989 (1989-06-27) * colonne 1, ligne 7 - colonne 4, ligne 32; figures 1-4 *<br>--- | 1-19 | |
| A | FR 2 692 979 A (BOSCH GMBH ROBERT) 31 décembre 1993 (1993-12-31) * page 4, ligne 16 - page 6, ligne 19; figure 1 *<br>--- | 1-19 | |
| A | US 6 234 654 B1 (KITAHARA TAKAHIDE ET AL) 22 mai 2001 (2001-05-22) * colonne 3, ligne 5-60; figures 1,2 *<br>--- | 1-19 | **DOMAINES TECHNIQUES RECHERCHES** (Int.Cl.7)<br><br>G01B<br>G01D |
| A | US 2002/115944 A1 (BARAK GILAD ET AL) 22 août 2002 (2002-08-22) * alinéas [0069]-[0113]; figures 1-8 *<br>----- | 1-19 | |

Le présent rapport a été établi pour toutes les revendications

| Lieu de la recherche | Date d'achèvement de la recherche | Examinateur |
|---|---|---|
| MUNICH | 29 avril 2004 | Beyfuß, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...................................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 04 07 5045

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-04-2004

| Document brevet cité au rapport de recherche | | Date de publication | Membre(s) de la famille de brevet(s) | | Date de publication |
|---|---|---|---|---|---|
| DE 4114398 | A | 29-10-1992 | DE | 4114398 A1 | 29-10-1992 |
| US 4665361 | A | 12-05-1987 | EP | 0124731 A1 | 14-11-1984 |
| US 4843259 | A | 27-06-1989 | CH | 672383 A5 | 15-11-1989 |
| | | | BE | 1000173 A6 | 05-07-1988 |
| | | | DE | 3734177 A1 | 05-05-1988 |
| | | | FR | 2606163 A1 | 06-05-1988 |
| | | | GB | 2197076 A ,B | 11-05-1988 |
| | | | IT | 1222994 B | 12-09-1990 |
| | | | NL | 8702575 A | 16-05-1988 |
| FR 2692979 | A | 31-12-1993 | DE | 4220801 A1 | 05-01-1994 |
| | | | FR | 2692979 A1 | 31-12-1993 |
| | | | JP | 6072118 A | 15-03-1994 |
| US 6234654 | B1 | 22-05-2001 | JP | 11304407 A | 05-11-1999 |
| | | | DE | 19918404 A1 | 28-10-1999 |
| US 2002115944 | A1 | 22-08-2002 | US | 2002101232 A1 | 01-08-2002 |
| | | | US | 6245109 B1 | 12-06-2001 |
| | | | AU | 1410201 A | 30-05-2001 |
| | | | WO | 0135872 A1 | 25-05-2001 |

EPO FORM P0460

Pour tout renseignement concernant cette annexe : voir Journal Officiel de l'Office européen des brevets, No.12/82